(19) Europäisches Patentamt

European Patent Office

Office européen des brevets

(11) **EP 3 457 844 B1**

(12) **EUROPEAN PATENT SPECIFICATION**

(45) Date of publication and mention
of the grant of the patent:
**16.11.2022  Bulletin 2022/46**

(51) International Patent Classification (IPC):
***A01N 1/02*** (2006.01)     ***C12N 5/071*** (2010.01)
***C12M 1/42*** (2006.01)     ***C12M 3/00*** (2006.01)

(21) Application number: **17728911.3**

(22) Date of filing: **22.05.2017**

(52) Cooperative Patent Classification (CPC):
**A01N 1/0242; A01N 1/0263; A01N 1/0278;
C12M 21/08; C12M 23/46; C12M 35/04;
C12N 5/0629**

(86) International application number:
**PCT/GB2017/000081**

(87) International publication number:
**WO 2017/198989 (23.11.2017 Gazette 2017/47)**

(54) **IMPROVEMENTS IN AND RELATING TO SKIN SAMPLE VIABILITY**

VERBESSERUNGEN AN UND IM ZUSAMMENHANG MIT DER HAUTPROBENLEBENSFÄHIGKEIT

AMÉLIORATIONS APPORTÉES ET RELATIVES À LA VIABILITÉ D'ÉCHANTILLON DE PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.05.2016  GB 201608899**

(43) Date of publication of application:
**27.03.2019  Bulletin 2019/13**

(73) Proprietor: **The University of Dundee
Dundee DD1 4HN (GB)**

(72) Inventors:
• **HICKERSON, Robyn Patricia
Balmullo KY16 0AN (GB)**
• **CONNEELY, Michael John
Dundee DD2 2AY (GB)**

(74) Representative: **Gordon, Naoise Padhraic Edward
Black & Associates Limited
Office 2/1
211 Dumbarton Road
Glasgow G11 6AA (GB)**

(56) References cited:
**WO-A1-01/93771          US-A- 5 914 264
US-A1- 2010 323 438     US-A1- 2011 172 683**

US-A1- 2015 018 948

• NEELTJE A. COOLEN ET AL: "Development of an in vitro burn wound model", WOUND REPAIR AND REGENERATION., vol. 16, no. 4, 1 July 2008 (2008-07-01), pages 559-567, XP055405481, US ISSN: 1067-1927, DOI: 10.1111/j.1524-475X.2008.00403.x
• GEGOTEK AGNIESZKA ET AL: "The role of transcription factor Nrf2 in skin cells metabolism", ARCHIVES OF DERMATOLOGICAL RESEARCH, SPRINGER, INTERNATIONAL, BERLIN, DE, vol. 307, no. 5, 24 February 2015 (2015-02-24), pages 385-396, XP035486401, ISSN: 0340-3696, DOI: 10.1007/S00403-015-1554-2 [retrieved on 2015-02-24]
• WASHINGTON Y. SANCHEZ ET AL: "Analysis of the metabolic deterioration of ex vivo skin from ischemic necrosis through the imaging of intracellular NAD(P)H by multiphoton tomography and fluorescence lifetime imaging microscopy", JOURNAL OF BIOMEDICAL OPTICS, vol. 15, no. 4, 1 January 2010 (2010-01-01), page 046008, XP055405491, USA ISSN: 1083-3668, DOI: 10.1117/1.3466580

**EP 3 457 844 B1**

- **MICHAEL S CHIN ET AL: "In vivo acceleration of skin growth using a servo-controlled stretching device", TISSUE ENGINEERING: PART C, vol. 16, no. 3, 1 June 2010 (2010-06-01), pages 397-405, XP055405479, US ISSN: 1937-3384, DOI: 10.1089/ten.tec.2009.0185**

## Description

Introduction

The present invention relates to improvements in and relating to skin sample viability and in particular to improving the viability of skin samples which are used to study the biology of skin and to evaluate drug delivery, metabolism, toxicity and efficacy of therapeutics.

Background to the Invention

Skin is the strong outer covering of vertebrate animals. Other animal coverings such as the arthropod exoskeleton have a different developmental origin, structure and chemical composition.

In mammals, the skin is an organ made up of multiple layers of ectodermal tissue covering the underlying tissue, ligaments, bones and internal organs. It acts as a sensory organ, protects the body against pathogens, ultraviolet damage, excessive water loss, provides insulation, temperature regulation and produces vitamin D.

The thickness of skin varies from location to location on an organism. In humans for example the skin located under the eyes and around the eyelids is the thinnest skin in the body at 0.5 mm thick whereas the skin on the palms and the soles of the feet is 4 mm thick and is around 1.4 mm thick on the back.

Mammalian skin is composed of two primary layers, the epidermis and the dermis. The epidermis is a stratified, cornified epithelium with specific barrier functions which is supported by the complex extracellular matrix environment of the dermis. In order for skin to retain its normal appearance and to function fully in a normal manner, both layers of the skin need to be present.

The epidermis forms a protective barrier over the body's surface, is responsible for keeping water in the body, protecting from UV light and preventing pathogens from entering.

The epidermis contains no blood vessels and cells in the deepest layers are nourished by diffusion from blood capillaries extending to the upper layers of the dermis.

The dermis comprises connective tissue and cushions the body from stress and strain. The dermis provides tensile strength and elasticity to the skin through an extracellular matrix composed of collagen fibrils, microfibrils, and elastic fibers. The dermis is tightly connected to the epidermis through a basement membrane and is structurally divided into two areas: a superficial area adjacent to the epidermis, called the papillary region, and a deep thicker area known as the reticular region.

Samples of skin may be removed from an animal body for the purpose of analysis or in order to grow a sample of skin where a skin graft is required. Skin sample types commonly used include human, porcine and murine skin. Skin grafting is an essential component of reconstructive surgery after burns, trauma, tumor excision, and correction of congenital anomalies. The best possible skin available for grafting is skin from the same patient taken from a donor site elsewhere on the body which is referred to as an autograft. Suitable skin graft donor sites are limited by body surface area and may also be affected by previous graft harvest or trauma. In patients suffering from large burns with limited donor skin sites, cadaver allografts are commonly used for temporary skin coverage.

In all cases, there is a need to maintain the skin sample in a healthy state and to slow or completely arrest deterioration of the quality of the sample whilst it is being stored.

Reliable skin models which recapitulate the features of live skin are essential for the investigation of cutaneous biology and drug discovery. Full-thickness ex vivo skin culture systems have been used extensively. The global market for ex vivo skin models includes academic researcher and the cosmetic industry with regards to safety and efficacy assessment for the increasing number of products developed for topical application. Within a global in vitro toxicity market expected to reach $27 billion by 2021, there is a growing demand for ex vivo skin models. Furthermore, the development of such a model system is of an increasing priority due to the European Community regulation that bans the use of animal testing for cosmetic ingredients.

US 2015/0018948 A1 discloses devices and methods useful for culturing, conditioning and/or stretching of tissues, wherein the tissue is placed on an expandable membrane. WO 01/93771 A1 describes a tissue expansion device, which may comprise electric motors for supplying alternating levels of force to a set of clamps, and sensors to measure forces and stretching distances. Further tissue stretching devices are disclosed in US 2011/0172683 A1 and US 5,914,264. US 2010/0323438 A1 discloses a method for culturing and conditioning cells on a sample such as skin, wherein a selected load is applied to the sample.

## Summary of the Invention

In accordance with the invention there is provided a method as defined in claim 1.

In the present application, ex vivo means any process performed outside the living organism and includes in vitro processes which occur in a test tube, culture dish or the like and explant processes where living cells, tissues, or organs are transferred from animals to a nutrient media.

The values of skin tension comprise a continuous range.

Preferably, the step of applying tension comprises stretching the skin sample and holding it in position upon the skin sample holder.

Preferably, the step of measuring the tension comprises applying a measurable force to the surface of a skin sample which is held in the skin sample holder.

Preferably, the force is applied by contacting a probe with the surface of the skin sample displacing the skin a predetermined amount so as to stretch the skin, wherein the amount of force for a given displacement provides a measure of skin tension.

Preferably, where an annular skin sample holder is used, a correction factor is applied to the force measurement to account for the effect of the radius of the skin sample holder.

Preferably, the step of measuring the ability of the first or other sample to maintain live skin characteristics comprises, wounding the skin and measuring keratin 17 expression as an indication of tissue viability.

Preferably, the step of wounding the skin is conducted using a device which ensures that wounds have a consistent depth and coverage across the skin.

Preferably, the step of wounding the skin uses a laser source wound the skin. Preferably, the step of measuring the ability of the first or other sample to maintain live skin cellular characteristics comprises measuring NRF2 activation in response to small molecules by measuring NQO1 mRNA levels.

Preferably, the step of measuring the ability of the first or other sample to maintain live skin mitochondrial characteristics comprises measuring mitochondrial metabolic activity.

Preferably, the skin sample holder comprises a culture dish with a skin sample holder which holds the skin sample and applies tension to the skin tissue sample.

Preferably, the skin sample holder is adapted to apply different levels of tension to the skin sample.

Preferably, the skin sample holder comprises a base frame, with a skin sample receiving surface upon which at least part skin sample may be placed and which extends across an area defined by the shape of the frame; and
a securing member which is releasably connectable to the base frame and a grip which holds the skin sample under tension.

Preferably, the skin tissue type is porcine, murine or human.

Preferably, for porcine and human skin, the one or more value of skin tension comprises a range of 0.22 to 0.57 N, assuming a specific and constant diameter and deflection distance.

More preferably, the range comprises 0.29 to 0.46 N, assuming a specific and constant diameter and deflection distance.

A skin sample holder may be provided for holding an ex vivo skin sample under tension, the skin sample tension being determined by the method of the present invention.

There may also be provided a method for measuring the tension in a skin sample culture apparatus in accordance with the method of the invention, the apparatus comprising:

a spacer which couples the force meter to the skin sample culture apparatus;
a probe which is extendable from the spacer for contact with a skin sample and which applies a force to the skin sample;
a force meter for measuring the force applied wherein the amount of force for a given displacement, defined by the spacer length, provides a measure of skin tension.

Preferably, the step of measuring the tension comprises applying a measurable force to the surface of a skin sample which is held in the skin sample holder.

Preferably, the force is applied by contacting a probe with the surface of the skin sample displacing the skin a predetermined amount so as to stretch the skin, wherein the amount of force for a given displacement provides a measure of skin tension.

Preferably, where an annular skin sample holder is used, a correction factor is applied to the force measurement to account for the effect of the radius of the skin sample holder.

Preferably, the probe further comprises computing means for calculating the correction factor.

For example, the computing means mean may be contained in the probe as hardware, firmware and/or software or may be a separate module or device.

Preferably, the probe is spherical.

Optionally, the probe is conical.

Optionally, the probe is cylindrical.

There is also provided a method of culturing a skin tissue sample at a tension determined by the method of the invention.

Preferably, the skin tissue type is porcine, murine or human and is cultured at a tension in the range 0.22 to 0.57 N, assuming a specific and constant diameter and deflection distance. More preferably, the range comprises 0.29 to 0.46 N.

**Brief Description of the Drawings**

The present invention will now be described with reference to the accompanying drawings in which:

Figure 1 is a flow diagram illustrating an example of a method in accordance with the present invention;

Figure 2A is a perspective view of an example of a device used in the method of the present invention; figure 2B is a side view of the device of figure 2A;

Figure 3 is a side view of a force meter fitted with a spacer collar used to measure skin sample tension;

Figure 4 is a schematic representation of a skin tension measurement;

Figure 5 is a schematic representation of a skin tension measurement for a device having a first radius;

Figure 6 is a schematic representation of a skin tension measurement for a device having a second radius;

Figure 7 is a schematic representation of a skin tension measurement for a device having a third radius;

Figure 8 is an example of a calibration curve for correcting measured skin tension measurements with respect to device radius;

Figures 9a shows K17 levels in a large section of wounded skin without tension and Figures 9b shows K17 levels in a large section of wounded skin with tension;

Figure 10a to 10f show keratin 17 levels for a porcine skin sample under varying levels of tension for unwounded skin and figures 10g to 10p show keratin 17 levels for a porcine skin sample under varying levels of tension for wounded skin;

Figures 11a to figure 11c show keratin 17 levels for a human skin sample under varying levels of tension for wounded skin;

Figure 12a shows a collection of slides from which K17 levels have been determined for unwounded and wounded porcine skin, Figure 12b shows a collection of slides from which K17 levels have been determined for wounded human skin and Figure 12c is a graph which plots estimated keratin 17 levels against tension;

Figures 13a to 13c show unwounded skin samples which are in vivo, from an industry standard source and held under tension in accordance with an example of the method of the present invention and Figures 13d to 13f show laser wounded skin samples which are in vivo, from an industry standard source and held under tension in accordance with the method of the present invention;

Figure 14 is a graph which plots NQO1 mRNA levels as a measure of NRF2 activity indicating cellular viability in the skin; and

Figure 15 is a graph which plots mitochondrial metabolic activity for fresh skin, skin under tension in accordance with an example of the method of the present invention, skin under no tension and devitalised skin.

## Detailed Description of the Drawings

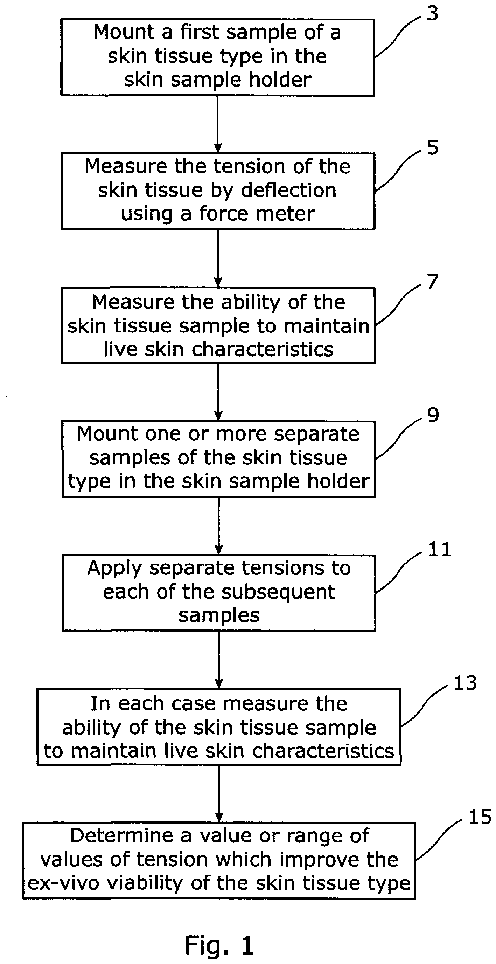

Figure 1 is a flow diagram 1 which illustrates an example of a method in accordance with the present invention. The method of the present invention allows ex vivo samples of a skin type, such as porcine, human or murine skin to be tested in order to determine preferred and/or optimum skin tension levels.

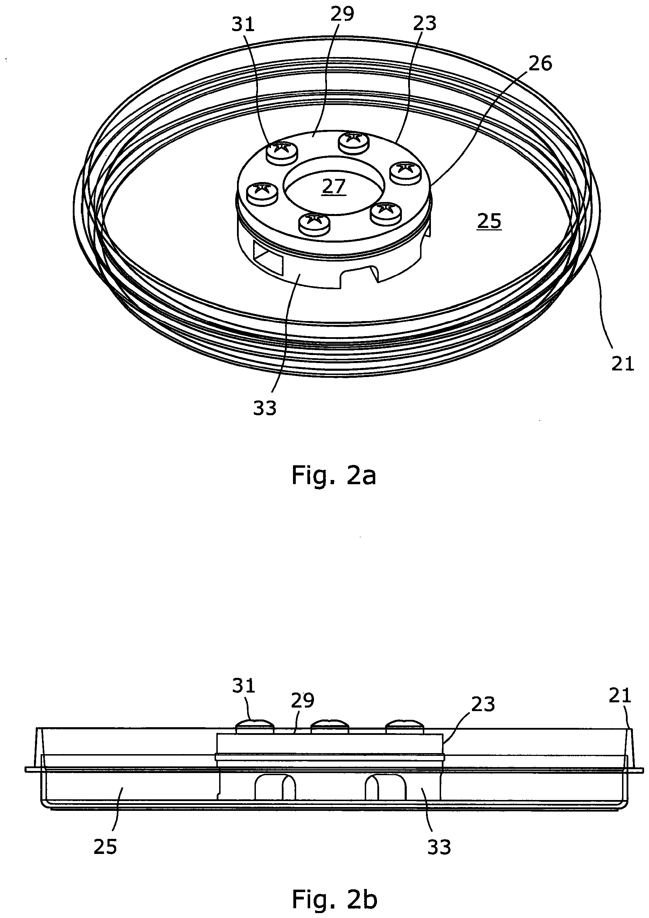

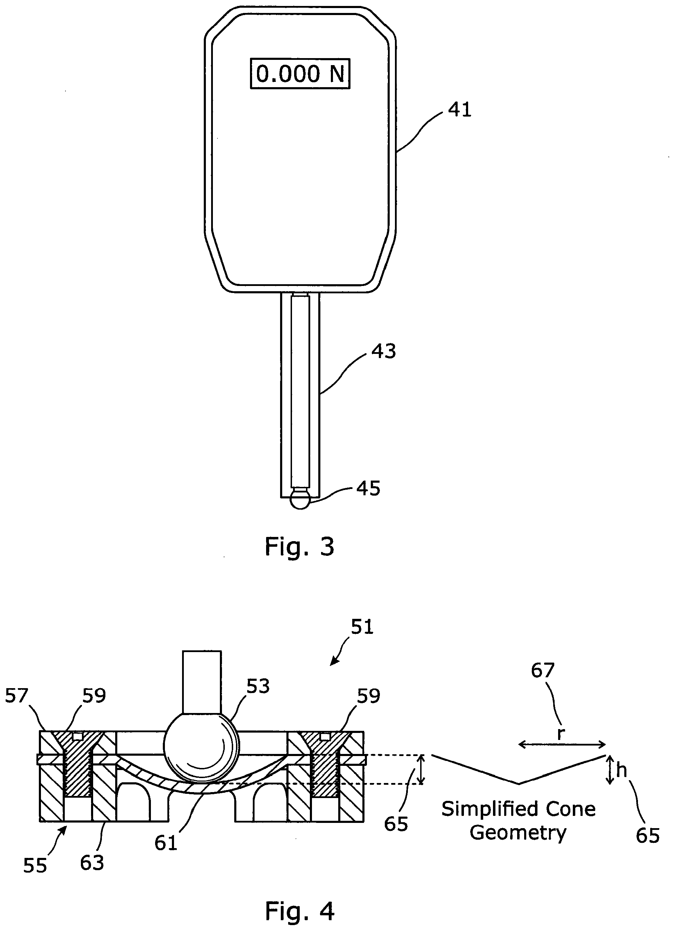

As shown in figure 1 a first sample of a skin tissue type is mounted 3 in a skin sample holder such as, for example, the device shown in figures 2A and 2B. Using the device of figures 2A and 2B, a tension is applied across the skin sample and measured 5 using a force meter or by other means. In this example of the present invention, the tension is measured using the force meter of figure 3.

The ability of the first sample to maintain live skin characteristics 7 is determined. In this example of the present invention, the live characteristics of the skin sample are measured by wounding the skin using a laser and measuring the excretion of keratin 17 (K17) in response to the wound as described in figures 3 to 12.

One or more separate samples of the skin tissue type are then separately mounted in the skin sample holder 9 and separate tensions are applied to each of the subsequent samples 11. In each case the ability of the skin tissue sample to maintain live skin characteristics is measured 13, such that, for the skin tissue type, a value or range of values of surface tension is determined which improve the in-vitro viability of the skin sample type.

The optimal tension window can then be determined based on the ability to respond to wounding (15). It will be appreciated that the method described above shows sample measurements being made one after another, such measurements may be made at the same time using separate skin sample holders for each sample.

Figure 2A is a perspective view of an example of a device used in the method of the present invention and figure 2B is a side view of the device of figure 2A. A skin culture dish 21 is shown containing a culture medium 25. The device 23 has a base 33 upon which a skin sample 27 is placed in use, and a cap 29 which is placed on top of a skin sample, the skin extending to the outer circumference 26 of the cap 29 and base 33. Fixings 31 extend through holes in the cap 29 and corresponding holes in the base 33 and are used to attach the cap 29 to the base 33. The fixings provide for the removable attachment of the cap 29 to the base 33. In use, the skin sample 27 is stretched across the base and secured to the base 33 and cap 29 using fixings 31.

Figure 3 shows an apparatus for measuring the tension in a skin sample which has been placed in the device of figures 2a and 2b. The apparatus comprises a force meter 41, a spacing collar 43 which slides over the probe shaft which extends downwards from the body of the force meter 41. The collar 43 is a cylindrical tube with a diameter and circumferences which matches that of the cap such that the end of the collar rests upon the cap 57 of the device of figure 4. The spherical indenter 45 exerts a force upon the skin sample.

Figure 4 is a schematic representation 51 of a skin tension measurement. It shows a spherical indenter 53, a skin sample culture device 55 similar to that of figures 2a and 2b which has a cap 57, countersunk fixings 59 which extend through the cap 57 into the base 63 to hold a skin sample in place and under tension. Both the cap and the base comprise annuli of substantially identical inner and outer radius. The radius of the cap 67 as measured at the inner diameter and the vertical displacement 65 are also shown. When a known displacement h 65, is exerted upon the skin sample 61, a force reading is recorded. This provides a measure of skin tension.

In cases where the device is annular, devices having different radii may be used.

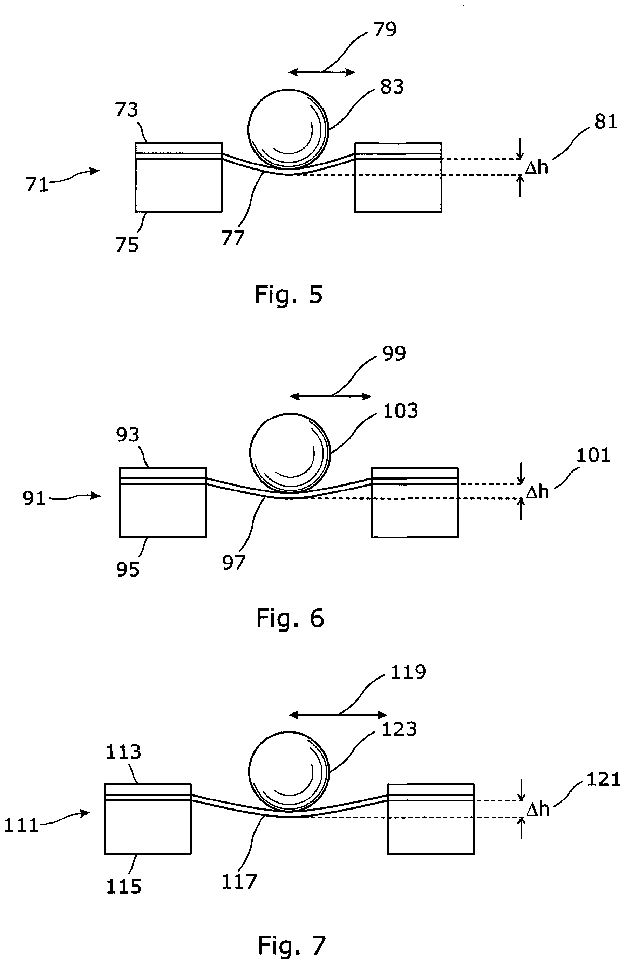

Figure 5 is a schematic representation of a skin tension measurement for a device having size 71 with a first radius 79, a cap 73, base 75. The vertical displacement 81 of the skin sample 77 as caused by the indenter 83 is shown.

Figure 6 is a schematic representation of a skin tension measurement for a device having size 91 with a first radius 99, a cap 93, base 95. The vertical displacement 101 of the skin sample 97 as caused by the indenter 103 is shown.

Figure 7 is a schematic representation of a skin tension measurement for a device having size 111 with a first radius 119, a cap 113, base 115. The vertical displacement 121 of the skin sample 117 as caused by the indenter 123 is shown.

Where the radii are different as shown in figures 5 to 7, it is necessary to account for the effect of the radius on the measured value of tension.

The elastic modulus E of the membrane mounted in the culture device, defined as the relationship between stress (force per unit area) and strain (proportional deformation), will be used to relate tension measurements in devices of different diameters using variable indentation distances.

In essence when the membrane is mounted in the culture device at the correct tension it will possess a certain elastic modulus.

It is assumed the probe is in frictionless contact and for simplicity that the deformed membrane conforms to a conical geometry with a uniform strain. It is also assumed the membrane is a linearly elastic material.

The probe applies an average stress over the membrane given by,

$$\sigma = \frac{F}{A_{CS}}$$

Where F is the normal force applied by the probe and $A_{CS}$ is the cross sectional area of the membrane.

Due to this stress, the membrane deforms to a ~conical geometry with a surface area defined as,

$$A_{cone} = \pi r \sqrt{r^2 + h^2}$$

Where r is the membrane diameter and h is the indentation distance, or equally the height of the cone formed by the stretched membrane. The area strain $\varepsilon$ over the stretched membrane is given by,

$$\varepsilon = \frac{\Delta A}{A_0}$$

Where $\Delta A$, the change in area of the membrane, is given by,

$$\Delta A = A_{cone} - A_0$$

$$\Delta A = \pi r \sqrt{r^2 + h^2} - \pi r^2$$

Therefore $\varepsilon$ can be written as,

$$\varepsilon = \frac{\sqrt{r^2 + h^2} - r}{r}$$

The elastic modulus E can now be fully defined as,

$$E = \frac{\sigma}{\varepsilon}$$

In examples of the present invention described herein the skin sample holder has a 15 mm diameter culture device with a 3 mm indentation/push depth (15-3) to characterise the optimum tension $F_0$ required in our membrane.

Therefore this will be our reference from which the conversion factors will be calculated, however, this approach can be applied generally to any diameter/depth values used for membrane characterisation.

To find the required probe force reading $F_x$ on a different diameter device we equate the elastic moduli.

$$E_0 = E_x$$

$$\frac{\sigma_0}{\varepsilon_0} = \frac{\sigma_x}{\varepsilon_x}$$

$$\frac{F_0/A_{CS_0}}{\varepsilon_0} = \frac{F_x/A_{CS_x}}{\varepsilon_x}$$

$$F_0 = \frac{F_x A_{CS_0} \varepsilon_0}{A_{CS_x} \varepsilon_x}$$

Using the values for our membrane characterisation (15-3) we can generate a matrix of conversion/correction factors to allow testing of devices of different diameters and also using different indentation depths/distances.

The correction factor (A) relates the measured force as follows,

$$F_{15-3} = \lambda F_{x-y}$$

Here, we see that, as expected, the correction factor for a 15 mm diameter device with an indentation depth of 3 mm is unity or 1.

Maintaining the device diameter at 15 mm but increasing indentation depth we see the factor decrease which is logical considering the membrane is stretched to a greater extent with an increase indentation. Similarly, with a smaller diameter device/membrane the correction factor decreases for a given indentation depth.

Due to the assumption of a linearly elastic material it is advised that strain is below 10% during tension measurements, i.e. the membrane is not stretched by an amount greater than 10% by the probe.

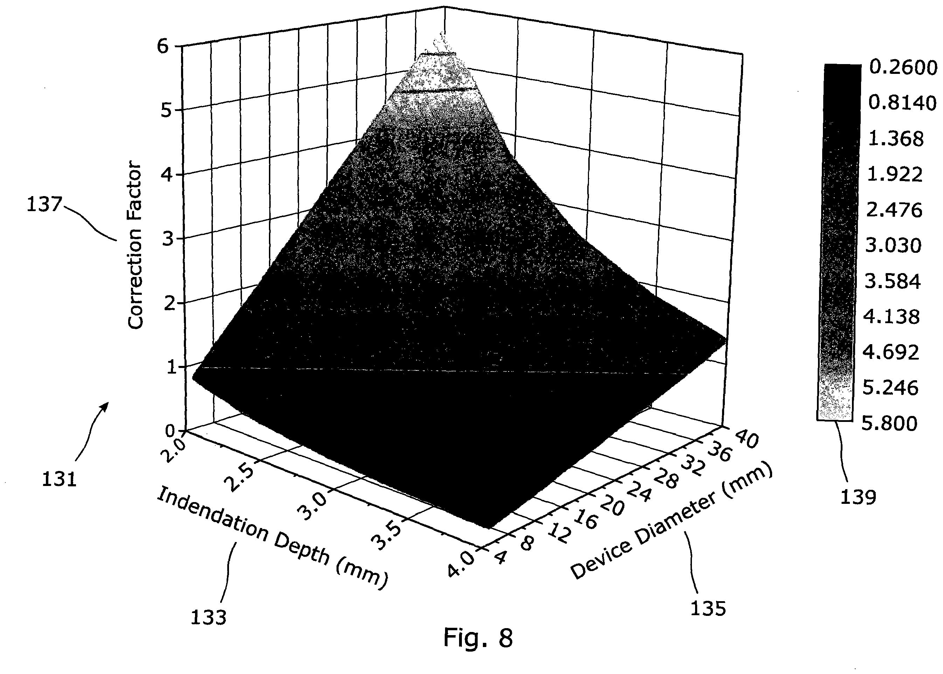

Figure 8 shows a 3D surface plot 131 of a section of the calculated conversion-correction factor matrix. Correction factors 137 are related to indentation depths 133 and device diameter 135 with the use of a colour scale 139.

Experimental results

Defatted skin was cultured at a user defined tension in the device of figure 2. The tension experienced across the surface of the skin sample was measured through an indentation measurement using the force meter of figure 3. Force was applied perpendicular to the surface of the skin and the skin tissue was stretched by a user-defined fixed distance (in this instance 3 mm).

In this example, the process for ensuring skin was mounted at a specific tension was an iterative process which required the skin sample to be fully mounted in the culture device before measuring the tension of the sample. Following mounting, the force meter 41 of figure 3, fitted with a spherical probe 45 and a spacer collar 43 to fix the indentation distance, was placed centrally over the skin sample holder. The spacing collar is put into firm contact with the top of the culture device, pushing the probe into contact with the skin, thus displacing it a defined distance. A record of the peak force imparted on the probe is then taken as a measure of tension. In order to fine tune the tension, the culture device was partially disassembled by loosening the screw fixings allowing small adjustments to the skin to be made. Tension was regulated to a relatively high degree of accuracy using this method.

In order to determine the manner in which ex vivo viability of a skin tissue type might be improved, measurements of the ability of the skin sample to maintain live skin characteristics such as metabolic activity, response to small molecule drugs, and the ability to respond to injury were made.

1. Tissue viability (keratin 17 expression following wounding)

Human and porcine skin was cultured under a range of tensions (i.e., no tension up to fully stretched) for 24-48 hours before being exposed to wounding using an Er-YAG laser set to a constant ablation depth and coverage. After 48 h, the skin was harvested, formalin fixed and stained for keratin 17 expression, which is a marker of the ability of skin to respond to wounding. A healthy skin sample which exhibits live skin characteristics would be expected to express keratin 17 in response to wounding. In the following pictures, K17 expression is shown by the white and/or lighter shaded areas in the slide.

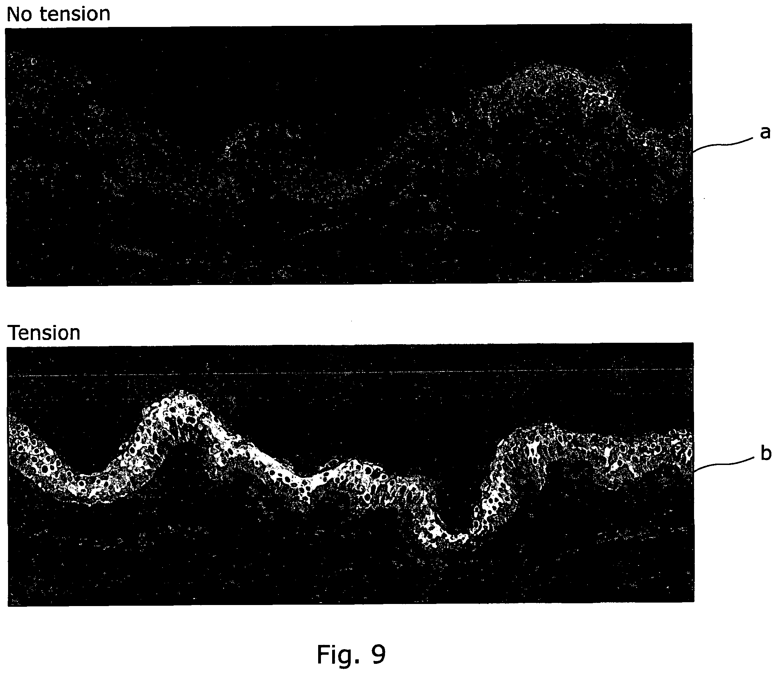

Figure 9 shows keratin 17 levels in porcine skin with and without tension. Figure 9a shows that keratin 17 expression is observed near the site of wounding when the skin is cultured without tension. Figure 9b shows that keratin 17 expression at consistent levels throughout the skin sample that is cultured at tension. The results show that the unwounded skin expresses little or no Keratin 17 until the skin tension reaches a level at which the skin is damaged by the stretch itself in figure 9f. In the case of healthy wounded skin, the expectation would be that keratin 17 expression would occur.

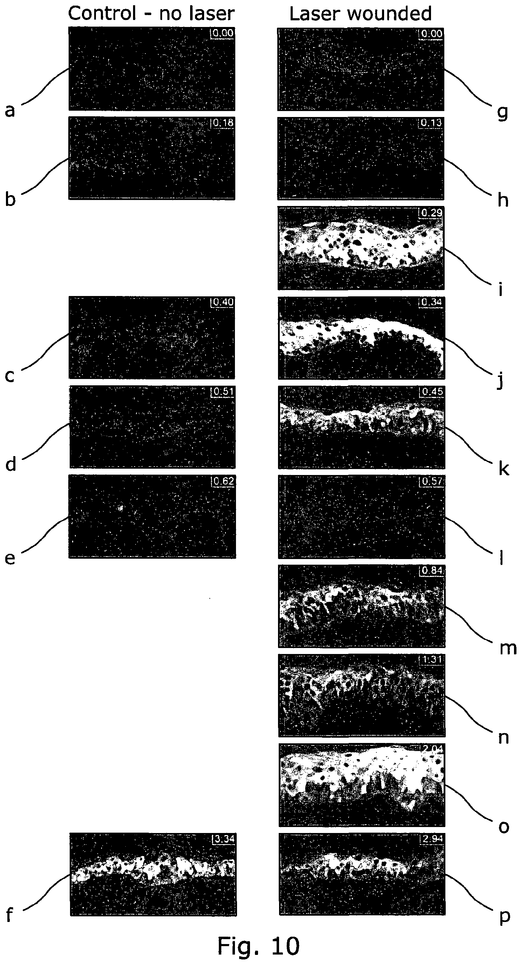

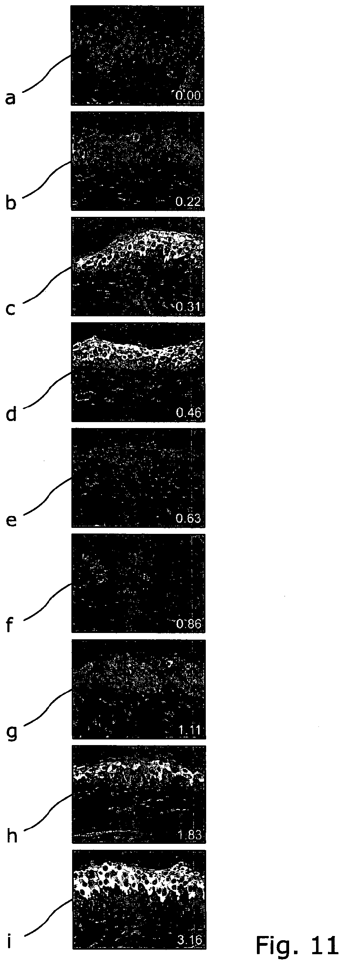

For figures 10 and 11, images were taken of skin tissue between the laser wounds. Figures 10a to 10f show keratin 17 levels for an unwounded skin sample under varying levels of tension and figures 10g to 10p show keratin 17 levels for a wounded skin sample under varying levels of tension . In each image the skin tension (in newtons; N) is shown in the top right hand corner of the image.

Figures 10g and 10h show skin samples with no tension and tension of 0.13 N respectively. At these tension levels, there is little or no keratin 17 expression. Keratin 17 expression is observed in figures 10i, 10j and 10k which are for skin samples with tensions of 0.29 N, 0.34 N and 0.45 N respectively. The largest apparent keratin 17 expression is seen at a tension of 0.29 N. Figure 10l shows little or no keratin 17 expression at a tension of 0.57 N. Based on these results, it can be concluded that there is an optimum range of tension values at which ex vivo skin samples should be held in order to maximise their viability. The range of certain viability is between 0.29 and 0.45 N for porcine skin. Skin cultured at a tension less than or equal to 0.13 or more than or greater than 0.57 N shows considerably reduced viability.

Figures 11a to 11i show keratin 17 levels for a wounded human skin sample prepared as described above under varying levels of tension. In each image the skin tension (in newtons; N) is shown in the bottom right hand corner of the image.

Figure 11a and 11b, show skin samples at no tension and tension of 0.22 N respectively. At these tension levels, there is little or no keratin 17 expression. Keratin 17 expression is observed in figures 11c and 11d which are for skin samples with tensions of 0.31 N and 0.46 N respectively. Figure 11e shows little or no keratin 17 expression at a tension of 0.63 N. Based on these results, it can be concluded that there is an optimum range of tension values at which ex vivo skin samples should be held in order to maximise their viability. The range of certain viability is between 0.31 and 0.46 N for human skin. Skin cultured at a tension less than or equal to 0.22 or greater than 0.63 N shows considerably reduced viability.

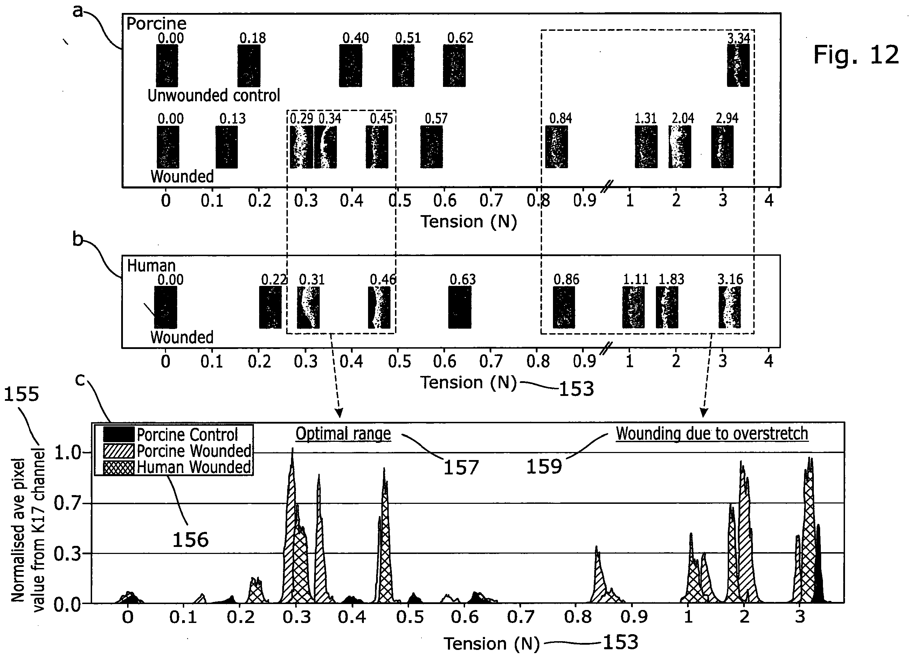

These findings are further supported by figure 12. Figure 12a shows a collection of slides from which K17 levels have been determined for unwounded and wounded porcine skin. Figure 12b shows a collection of slides from which K17 levels have been determined for wounded human skin. Figure 12c is a graph which plots estimated keratin 17 levels 155 as measured by the normalised average pixel value from the images against skin tension 153 for a porcine skin control sample, a wounded porcine skin sample and a wounded human skin sample. The key 156 provides information to distinguish between the results for the different samples used. The peak value is within the optimum range 157 shown in figures 10 and 11. The second peak is caused by keratin 17 which has been expressed due to damage caused by the skin being overstretched 159.

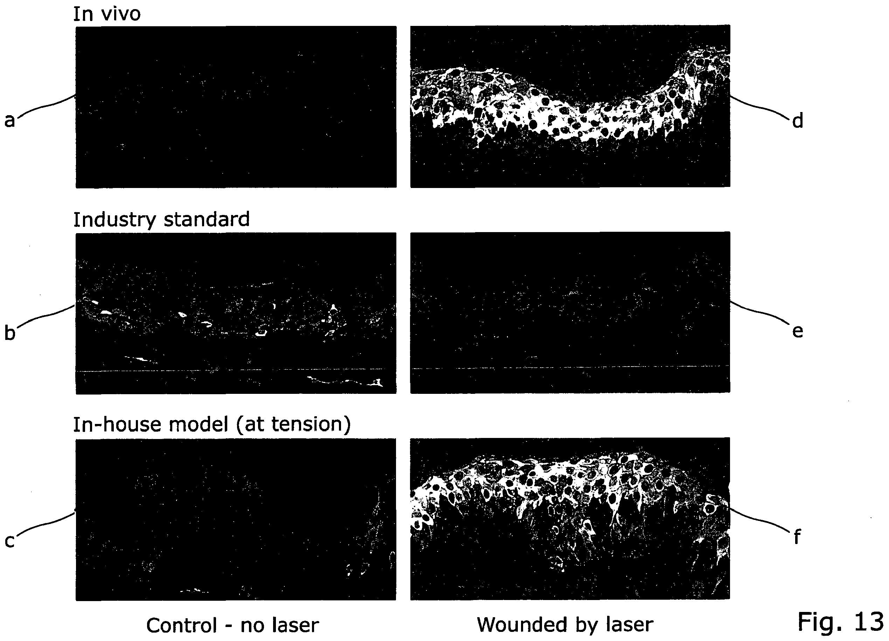

Figures 13a to 13c are pictures of unwounded skin samples which are, respectively, in vivo, from an industry standard source and held under tension in accordance with an example of the method of the present invention. Figures 13d to 13f are pictures of laser wounded skin samples which are, respectively, in vivo, from an industry standard source and held under tension in accordance with the method of the present invention. The in vivo skin was prepared from a biopsy taken from a human volunteer 24 h after wounding with the Er-YAG laser.

Staining shows uniform induction of K17 in the wounded in vivo skin. The human skin cultured at a level of tension at which the ex vivo skin sample's viability was improved or optimised in accordance with the method of the present invention showed a wound response similar to what is observed in vivo. This is clearly shown when figures 13 d and 13f are compared. The industry standard model did not show any response to wounding as shown in figure 13e.

2. Cellular viability (NQO1 expression)

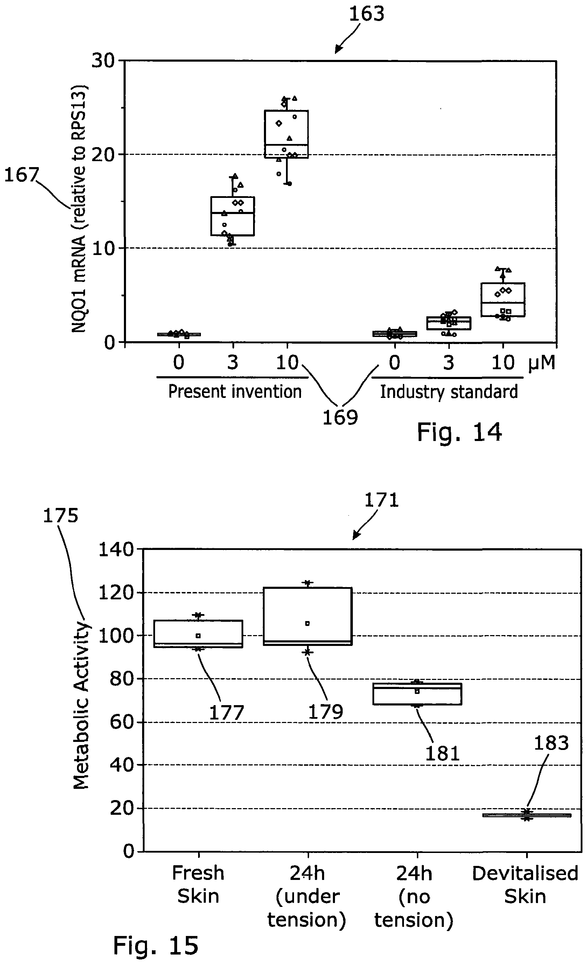

Figure 14 is a graph 163 which plots NQO1 mRNA levels upon treatment with an NRF2 activator. Figure 14 summarises an experiment in which skin cultured using the method of the present invention was treated with a small molecule NRF2 activator (TBE-31) in order to evaluate the intracellular viability of the tissue. NRF2 transcriptionally regulates multiple genes that play both direct and indirect roles in activating intracellular antioxidative pathways.

One of the genes that is upregulated upon NRF2 activation is NAD(P)H dehydrogenase [quinone] 1 (NQO1). Figure 14 is a graph which shows NQO1 mRNA levels 167 obtained from total RNA isolated from the skin treated with TBE-31 compound (a potent NRF2 activator) at the indicated concentrations 169. It is shown here that the cellular viability of the skin cultured in this device responds to NRF2 activators to a greater extent than does the industry standard.

3. Mitochondrial viability (MTT conversion assay)

Figure 15 is a graph 171 which plots metabolic activity 175 for fresh skin 177, skin under tension in accordance with an example of the method of the present invention 179, skin under no tension 181 and devitalised skin 183. The graph shows similar responses for fresh skin 177 and skin under tension in accordance with an example of the method of the present invention 179.

Improvements and modifications may be incorporated herein without deviating from the scope of the invention as defined in the appended claims.

**Claims**

1. A method for improving the ex vivo viability of a skin tissue type, the method comprising the steps of:

   mounting samples of the skin tissue type in a skin sample holder;
   applying separate tensions to at least some of the samples; and
   measuring the ability of the at least some of the samples to maintain live skin characteristics, such that, for the skin tissue type, the measurements of live skin characteristics determine values of skin tension which improve the ex vivo viability of the skin tissue type, wherein the values of skin tension comprise a continuous range of tension values and tension measurements are made in multiple samples of the skin tissue type to populate the range of tension values between unstretched and a maximum stretch.

2. A method as claimed in in any preceding claim wherein, the step of applying tension comprises stretching the skin sample and holding it in position upon the skin sample holder.

3. A method as claimed in any preceding claim wherein, the step of measuring the tension comprises applying a measurable force to the surface of the skin sample which is held in the skin sample holder.

4. A method as claimed in claim 3 wherein, the force is applied by contacting a probe with the surface of the skin sample displacing the skin a predetermined amount so as to stretch the skin, wherein the amount of force for a given displacement provides a measure of skin tension.

5. A method as claimed in claim 3 or claim 4 wherein, an annular skin sample holder is used, and a correction factor is applied to the force measurement to account for the effect of the radius of the skin sample holder.

6. A method as claimed in any preceding claim wherein, the step of measuring the ability of the first or other sample to maintain live skin characteristics comprises, wounding the skin and measuring keratin 17 expression as an indication of tissue viability.

7. A method as claimed in claim 6 wherein, the step of wounding the skin is conducted using a device which ensures that wounds have a consistent depth and coverage across the skin.

8. A method as claimed in claim 6 or claim 7 wherein, the step of wounding the skin uses a laser source to wound the skin.

9. A method as claimed in any preceding claim wherein, the step of measuring the ability of the first or other sample to maintain live skin cellular characteristics comprises measuring NRF2 activation in response to small molecules by measuring NQ01 mRNA levels.

10. A method as claimed in any preceding claim wherein the skin sample holder is mounted in a culture dish and applies tension to the skin tissue sample.

11. A method as claimed in any preceding claim, further comprising culturing a skin tissue sample at a tension determined by the methoc of claims 1-10.

12. A method as claimed in claim 11 wherein, for porcine and human skin, the skin tissue sample is cultured at a tension in the range of 0.22 to 0.57 N, assuming a specific and constant diameter and deflection distance.

13. A method as claimed in claim 12 wherein, the range comprises 0.29 to 0.46 N, for a given skin sample holder diameter and deflection distance.

**Patentansprüche**

1. Verfahren zur Verbesserung der Ex-vivo-Lebensfähigkeit eines Hautgewebetyps, wobei das Verfahren die folgenden Schritte umfasst:

Anbringen von Proben des Hautgewebetyps in einem Hautprobenhalter;
Anwenden separater Spannungen auf mindestens einige der Proben; und
Messen der Fähigkeit von den mindestens einigen der Proben, um Eigenschaften von lebender Haut beizubehalten, sodass für den Hautgewebetyp die Messungen von Eigenschaften von lebender Haut Werte der Hautspannung bestimmen, die die Ex-vivo-Lebensfähigkeit des Hautgewebetyps verbessern, wobei die Werte der Hautspannung einen kontinuierlichen Bereich von Spannungswerten umfassen und Spannungsmessungen in mehreren Proben des Hautgewebetyps vorgenommen werden, um den Bereich von Spannungswerten zwischen ungedehnt und einer maximalen Dehnung zu befüllen.

2. Verfahren nach einem vorhergehenden Anspruch, wobei der Schritt des Anwendens von Spannung das Dehnen der Hautprobe und das Halten derselben in Position auf dem Hautprobenhalter umfasst.

3. Verfahren nach einem vorhergehenden Anspruch, wobei der Schritt des Messens der Spannung das Anwenden einer messbaren Kraft auf die Oberfläche der Hautprobe, die in dem Hautprobenhalter gehalten wird, umfasst.

4. Verfahren nach Anspruch 3, wobei die Kraft angewendet wird, indem eine Sonde mit der Oberfläche der Hautprobe in Kontakt gebracht wird, wodurch die Haut um einen vorbestimmten Betrag verschoben wird, um die Haut zu dehnen, wobei die Menge an Kraft für eine gegebene Verschiebung ein Maß für die Hautspannung bereitstellt.

5. Verfahren nach Anspruch 3 oder Anspruch 4, wobei ein ringförmiger Hautprobenhalter verwendet wird und ein Korrekturfaktor auf die Kraftmessung angewendet wird, um die Wirkung des Radius des Hautprobenhalters zu berücksichtigen.

6. Verfahren nach einem vorhergehenden Anspruch, wobei der Schritt des Messens der Fähigkeit der ersten oder der anderen Probe, um Eigenschaften von lebender Haut beizubehalten, das Verwunden der Haut und das Messen der Keratin-17-Expression als Hinweis auf die Lebensfähigkeit des Gewebes umfasst.

7. Verfahren nach Anspruch 6, wobei der Schritt des Verwundens der Haut unter Verwendung einer Vorrichtung durchgeführt wird, die sicherstellt, dass Wunden eine konsistente Tiefe und Abdeckung über die Haut aufweisen.

8. Verfahren nach Anspruch 6 oder Anspruch 7, wobei der Schritt des Verwundens der Haut eine Laserquelle verwendet, um die Haut zu verwunden.

9. Verfahren nach einem vorhergehenden Anspruch, wobei der Schritt des Messens der Fähigkeit der ersten oder der anderen Probe, zelluläre Eigenschaften von lebender Haut beizubehalten, das Messen der NRF2-Aktivierung als Antwort auf kleine Moleküle durch Messen der NQO1-mRNA-Pegel umfasst.

10. Verfahren nach einem vorhergehenden Anspruch, wobei der Hautprobenhalter in einer Kulturschale angebracht wird und Spannung auf die Hautprobe anwendet.

11. Verfahren nach einem vorhergehenden Anspruch, ferner umfassend das Kultivieren einer Hautgewebeprobe bei einer Spannung, die durch das Verfahren der Ansprüche 1-10 bestimmt wird.

12. Verfahren nach Anspruch 11, wobei für Schweine- und Menschenhaut die Hautgewebeprobe bei einer Spannung in dem Bereich von 0,22 bis 0,57 N kultiviert wird, wobei ein spezifischer und konstanter Durchmesser und eine Ablenkungsstrecke angenommen werden.

13. Verfahren nach Anspruch 12, wobei der Bereich 0,29 bis 0,46 N für einen gegebenen Durchmesser des Hautprobenhalters und eine gegebene Ablenkungsstrecke umfasst.

**Revendications**

1. Procédé d'amélioration de la viabilité ex vivo d'un type de tissu cutané, le procédé comprenant les étapes consistant à :

> monter des échantillons du type de tissu cutané dans un porte-échantillons de peau ;
> appliquer des tensions distinctes sur au moins certains des échantillons ; et
> mesurer la capacité des au moins certains des échantillons à maintenir des caractéristiques de peau vivante, de telle sorte que, pour le type de tissu cutané, les mesures de caractéristiques de peau vivante déterminent des valeurs de tension de peau qui améliorent la viabilité ex vivo du type de tissu cutané, les valeurs de tension de peau comprenant une plage continue de valeurs de tension et des mesures de tension étant effectuées dans de multiples échantillons du type de tissu cutané pour remplir la plage de valeurs de tension entre un étirement non étiré et un étirement maximal.

2. Procédé tel que revendiqué dans n'importe quelle revendication précédente, dans lequel l'étape consistant à appliquer une tension comprend l'étirement de l'échantillon de peau et le fait de le maintenir en position sur le porte-échantillons de peau.

3. Procédé tel que revendiqué dans n'importe quelle revendication précédente, dans lequel l'étape consistant à mesurer la tension comprend l'application d'une force mesurable sur la surface de l'échantillon de peau qui est maintenu dans le porte-échantillons de peau.

4. Procédé tel que revendiqué dans la revendication 3, dans lequel la force est appliquée par mise en contact d'une sonde avec la surface de l'échantillon de peau, déplaçant la peau d'une quantité prédéterminée de façon à étirer la peau, la quantité de force pour un déplacement donné fournissant une mesure de tension de peau.

5. Procédé tel que revendiqué dans la revendication 3 ou la revendication 4, dans lequel un porte-échantillons de peau annulaire est utilisé, et un facteur de correction est appliqué sur la mesure de force pour tenir compte de l'effet du rayon du porte-échantillons de peau.

6. Procédé tel que revendiqué dans n'importe quelle revendication précédente, dans lequel l'étape consistant à mesurer la capacité du premier échantillon ou d'un autre échantillon à maintenir des caractéristiques de peau vivante comprend le fait de blesser la peau et de mesurer l'expression de la kératine 17 en tant qu'indication de la viabilité des tissus.

7. Procédé tel que revendiqué dans la revendication 6, dans lequel l'étape consistant à blesser la peau est menée à l'aide d'un dispositif qui garantit que des plaies ont une profondeur et une couverture cohérentes sur toute la peau.

8. Procédé tel que revendiqué dans la revendication 6 ou la revendication 7, dans lequel l'étape consistant à blesser la peau utilise une source laser pour blesser la peau.

9. Procédé tel que revendiqué dans n'importe quelle revendication précédente, dans lequel l'étape consistant à mesurer la capacité du premier échantillon ou d'un autre échantillon à conserver des caractéristiques cellulaires de peau vivante comprend le fait de mesurer l'activation NRF2 en réponse à de petites molécules par mesure des niveaux d'ARNm NQO1.

10. Procédé tel que revendiqué dans n'importe quelle revendication précédente dans lequel le porte-échantillons de peau est monté dans une boîte pour culture et applique une tension sur l'échantillon de tissu cutané.

11. Procédé tel que revendiqué dans n'importe quelle revendication précédente, comprenant en outre la culture d'un échantillon de tissu cutané à une tension déterminée par le procédé des revendications 1 à 10.

12. Procédé tel que revendiqué dans la revendication 11, dans lequel, pour de la peau porcine et humaine, l'échantillon de tissu cutané est cultivé à une tension comprise dans la plage de 0,22 à 0,57 N, en supposant un diamètre et une distance de déviation spécifiques et constants.

13. Procédé tel que revendiqué dans la revendication 12, dans lequel la plage va de 0,29 à 0,46 N, pour un diamètre de porte-échantillons de peau et une distance de déviation donnés.

Fig. 1

Fig. 2a

Fig. 2b

**Fig. 3**

**Fig. 4**

Simplified Cone
Geometry

Fig. 5

Fig. 6

Fig. 7

Fig. 8

EP 3 457 844 B1

Fig. 9

Fig. 10

a    0.00

b    0.22

c    0.31

d    0.46

e    0.63

f    0.86

g    1.11

h    1.83

i    3.16

Fig. 11

Fig. 12

In vivo

Industry standard

In-house model (at tension)

Control - no laser                    Wounded by laser                    Fig. 13

EP 3 457 844 B1

Fig. 14

Fig. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150018948 A1
- WO 0193771 A1
- US 20110172683 A1
- US 5914264 A
- US 20100323438 A1